(19) Europäisches Patentamt
European Patent Office
Office européen des brevets

(11) Veröffentlichungsnummer: **0 141 072**
**A2**

(12) **EUROPÄISCHE PATENTANMELDUNG**

(21) Anmeldenummer: **84109291.9**

(22) Anmeldetag: **06.08.84**

(51) Int. Cl.4: **C 07 D 513/04**
A 61 K 31/425
//(C07D513/04, 285:00, 235:00)

(30) Priorität: **17.08.83 DE 3329621**

(43) Veröffentlichungstag der Anmeldung:
**15.05.85 Patentblatt 85/20**

(84) Benannte Vertragsstaaten:
**AT BE CH DE FR GB IT LI LU NL SE**

(71) Anmelder: **BAYER AG**
**Konzernverwaltung RP Patentabteilung**
**D-5090 Leverkusen 1 Bayerwerk(DE)**

(72) Erfinder: **Ingendoh, Axel, Dr.**
**Unterste Dillenberg 18**
**D-5620 Velbert 15(DE)**

(72) Erfinder: **Meyer, Horst, Dr.**
**Henselweg 11**
**D-5600 Wuppertal 1(DE)**

(72) Erfinder: **Garthoff, Bernward, Dr.**
**Händelstrasse 22**
**D-4010 Hilden(DE)**

(54) **Aminoalkyl-imidazothiadiazol-alkencarbonsäureamide, neue Zwischenprodukte zu ihrer Herstellung, ihre Herstellung und ihre Verwendung in Arzneimitteln.**

(57) Die vorliegende Erfindung betrifft neue 2- Aminoalkyl-imidazo -[2,1-b]-1,3,4- thiadiazol -5-yl- alkencarbonsäureamide, verschiedene Verfahren zu deren Herstellung, neue Zwischenprodukte, die bei der Herstellung eingesetzt werden, und die neuen Verbindungen enthaltende Arzneimittel, insbesondere Antihypertensiva, Diuretika und Uricosurica.

EP 0 141 072 A2

Croydon Printing Company Ltd.

BAYER AKTIENGESELLSCHAFT 5090 Leverkusen, Bayerwerk

Konzernverwaltung RP
Patentabteilung Sft/by-c

# Aminoalkyl-imidazothiadiazol-alkencarbonsäureamide, neue Zwischenprodukte zu ihrer Herstellung, ihre Herstellung und ihre Verwendung in Arzneimitteln

Die vorliegende Erfindung betrifft neue Aminoalkyl-imidazothiadiazol-alkencarbonsäureamide, verschiedene Verfahren zu deren Herstellung, neue Zwischenprodukte, die bei deren Herstellung eingesetzt werden, und die neuen Verbindungen enthaltende Arzneimittel, insbesondere Antihypertensiva, Diuretika und Uricosurica.

Gegenstand der Erfindung sind neue Amino-alkyl-imidazothiadiazol-alkencarbonsäureamide der allgemeinen Formel (I)

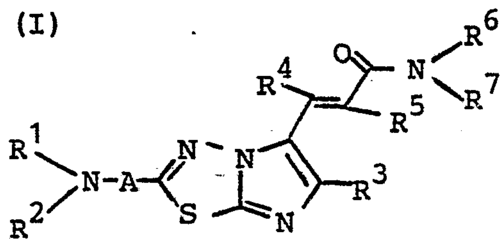

(I)

in welcher

$R^1$ Wasserstoff, Aryl oder einen aliphatischen Kohlenwasserstoffrest bedeutet, der gegebenenfalls durch O, S, N, N-Alkyl, NH, N-Aryl oder N-Aralkyl unterbrochen ist und der gegebenenfalls substituiert ist durch Hydroxy, Alkoxy, Alkyl, Trifluormethyl, Halogen, Phenyl, Alkoxycarbonyl oder Dialkylamino, wobei die beiden Alkylreste gegebenenfalls gemeinsam mit dem N-Atom einen 5- bis 7-gliedrigen Ring bilden, der gegebenenfalls durch ein Heteroatom aus der Gruppe O, S, NH oder N-Alkyl unterbrochen ist, und wobei diese vorgenannten Alkyl- und Phenylreste ihrerseits gegebenenfalls substituiert sind durch Halogen, Trifluormethyl, Alkyl, Aryl, Aralkyl, Alkoxy, Alkylmercapto oder $SO_2$-Alkyl, und

$R^2$ die für $R^1$ angegebene Bedeutung besitzt, wobei $R^2$ und $R^1$ gleich oder verschieden sein können, oder $R^2$ gemeinsam mit $R^1$ mit dem Stickstoffatom einen 3- bis 8-gliedrigen gesättigten oder ungesättigten Ring bildet, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl, Aryl oder Aralkyl substituiert ist, wobei dieser 3- bis 8-gliedrige Ring durch 1 bis 4 gleiche oder verschiedene Substituenten aus der Gruppe Alkyl, Halogen, Aryl, Aralkyl, Alkoxycarbonyl, Hydroxyalkyl, Alkoxyalkyl oder Trifluormethyl, substituiert sein kann, oder wobei dieser Ring mit einem aromatischen Ring kondensiert sein kann,

A für eine gegebenenfalls durch 1 oder 2 Heteroatome aus der Gruppe Sauerstoff, Schwefel und Stickstoff unterbrochenen Kette aus 1-4 Kohlenstoffatomen steht, die gesättigt oder ungesättigt oder Teil eines 3- bis 8-gliedrigen Ringes sein kann und bei der jedes Stickstoff- und Kohlenstoffatom durch Reste $R^1$ substituiert sein kann, welche gleich oder verschieden sein können,

$R^3$ die für $R^1$ angegebene Bedeutung besitzt und mit $R^1$ gleich oder verschieden ist, oder für Furyl, Phenyl, Thienyl, Pyrimidyl, Pyrazinyl, Chinolinyl, Isochinolinyl oder Pyridyl steht, wobei die Ringe gegebenenfalls substituiert sind durch 1, 2 oder 3 gleiche oder verschiedene Substituenten aus der Gruppe Alkyl, Aryl, Alkoxy, Halogen, Nitro, Trifluormethyl, $SO_n$-Alkyl (n = 0,1 oder 2) oder $NR^9R^{10}$, wobei $R^9$ und $R^{10}$ die oben angegebene Bedeutung von $R^1$ und $R^2$ besitzen,

$R^4$ für Wasserstoff, Trifluormethyl oder Alkyl steht,

$R^5$ für Wasserstoff, Alkyl, Cyano, Halogen, Nitro, $SO_n$-Alkyl (n = 0, 1 oder 2) oder $CXR^8$ steht, wobei X = O oder S bedeutet, und $R^8$ die oben angegebene Bedeutung von $R^1$ besitzt, und

$R^6$ und $R^7$ jeweils die für $R^1$ angegebene Bedeutung besitzen und mit $R^1$ gleich oder verschieden sind oder gemeinsam mit dem Stickstoffatom einen 3- bis 8-gliedrigen gesättigten oder ungesättigten Ring

bilden, der gegebenenfalls ein oder zwei weitere Heteroatome aus der Gruppe Sauerstoff, Schwefel oder Stickstoff enthält, wobei der Stickstoff gegebenenfalls durch Wasserstoff, Alkyl, Aryl oder Aralkyl substituiert ist, und wobei dieser 3- bis 8-gliedrige Ring gegebenenfalls durch 1, 2, 3 oder 4, bevorzugt 1 oder 2, gleiche oder verschiedene Substituenten aus der Gruppe Alkyl, Aryl, Aralkyl, Halogen, Hydroxy, Alkoxycarbonyl, Hydroxyalkyl, Alkoxyalkyl, Alkoxy oder Trifluormethyl substituiert ist und wobei dieser Ring mit einem gegebenenfalls durch ein bis drei dieser Substituenten substituierten aromatischen Ring kondensiert sein kann,

sowie ihre stereoisomeren Formen der verschiedenen Enantiomeren, Diastereomeren und E/Z-Isomeren sowie ihre pharmazeutisch unbedenklichen Säureadditionssalze.

In den obigen Substituentendefinitionen steht der Ausdruck "Aryl" für einen aromatischen Kohlenwasserstoffrest mit 6 bis 14 C-Atomen, bevorzugt 6 bis 10 C-Atomen, insbesondere für Phenyl oder Naphthyl.

Der Ausdruck "aliphatischer Kohlenwasserstoffrest" steht für einen geradkettigen, verzweigten, cyclischen, gesättigten oder ungesättigten Kohlenwasserstoffrest mit 1 bis 12 C-Atomen, insbesondere mit 1 bis 6 C-Atomen.

Der Ausdruck "Alkyl" steht für einen gesättigten, geradkettigen, verzweigten oder cyclischen Alkylrest mit 1 bis 10 C-Atomen, insbesondere mit 1 bis 6 C-Atomen.

Der Ausdruck "Aralkyl" steht für eine Alkylengruppe mit 1 bis 6 C-Atomen, vorzugsweise 1 bis 4 C-Atomen, die durch Aryl (wie oben definiert), vorzugsweise Phenyl oder Naphthyl substituiert ist.

Der Ausdruck "Alkoxy" steht für einen Alkoxyrest mit 1 bis 12 C-Atomen, insbesondere mit 1 bis 6 C-Atomen, der geradkettig oder verzweigt sein kann.

"Halogen" steht vorzugsweise für Fluor, Chlor oder Brom.

Gegenstand der vorliegenden Erfindung sind vorzugsweise Verbindungen der allgemeinen Formel (I), in welcher

$R^1$ Wasserstoff, Aryl oder einen aliphatischen Kohlenwasserstoffrest bedeutet, der gegebenenfalls durch O, S, N, N-Alkyl oder NH unterbrochen ist und der gegebenenfalls substituiert ist durch Hydroxy, Alkoxy, Alkyl, Trifluormethyl, Halogen, Alkoxycarbonyl oder Dialkylamino, wobei die beiden Alkylreste gegebenenfalls gemeinsam mit dem N-Atom einen 5- bis 7-gliedrigen Ring bilden, der gegebenenfalls durch ein Heteroatom aus der Gruppe O, S, NH oder N-Alkyl unterbrochen ist, und wobei diese vorgenannten Alkyl- und Phenylreste ihrerseits gegebenenfalls substituiert sind durch Halogen, Trifluormethyl, Alkyl, Alkoxy, Alkylmercapto oder $SO_2$-Alkyl, und

$R^2$ die für $R^1$ angegebene Bedeutung besitzt, wobei $R^2$ und $R^1$ gleich oder verschieden sein können, oder $R^2$ gemeinsam mit $R^1$ mit dem Stickstoffatom einen 5- bis 7-gliedrigen gesättigten oder ungesättigten Ring bildet, der gegebenenfalls 1 oder 2 weitere Heteroatome aus der Gruppe Sauerstoff, Schwefel oder Stickstoff enthält, wobei der Stickstoff gegebenenfalls durch Wasserstoff oder Alkyl substituiert ist, wobei dieser 5- bis 7-gliedrige Ring durch 1 bis 4 gleiche oder verschiedene Substituenten aus der Gruppe Alkyl, Halogen, Aryl, Aralkyl, Alkoxycarbonyl, Hydroxyalkyl, Alkoxyalkyl oder Trifluormethyl substituiert sein kann,

A für eine gegebenenfalls durch ein Heteroatom aus der Gruppe Sauerstoff, Schwefel und Stickstoff unterbrochene Kette aus 1-4 Kohlenstoffatomen steht, die gesättigt oder ungesättigt oder Teil eines 4- bis 7-gliedrigen Ringes sein kann und bei der jedes Stickstoff- und Kohlenstoffatom durch Reste $R^1$ substituiert sein kann, die gleich oder verschieden sein können,

$R^3$ für einen Phenyl-, Naphthyl-, Furyl-, Thienyl-, Pyrimidyl-, Pyrazinyl-, Chinolyl-, Isochinolyl- oder Pyridylrest steht, welcher gegebenenfalls substituiert ist durch 1 bis 3 gleiche oder verschiedene Substituenten aus der Gruppe Halogen, Nitro, Trifluormethyl, Alkyl, Alkoxy, Dialkylamino oder $SO_n$-Alkyl (n = 0, 1 oder 2), wobei die genannten Alkyl- und Alkoxyreste jeweils 1-4 Kohlenstoffatome enthalten,

$R^4$ für Wasserstoff, Trifluormethyl oder Alkyl steht,

$R^5$ für Wasserstoff, Alkyl, Cyano, Halogen oder $CXR^8$ steht, wobei X = O oder S bedeutet, und $R^8$ die oben angegebene Bedeutung von $R^1$ besitzt, und

$R^6$ und $R^7$ jeweils die für $R^1$ angegebene Bedeutung besitzen und mit $R^1$ gleich oder verschieden sind oder
gemeinsam mit dem Stickstoffatom einen 5- bis 7-gliedrigen gesättigten oder ungesättigten Ring bilden, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl mit 1 bis 4 Kohlenstoffatomen, Phenyl oder Benzyl substituiert ist,

sowie ihre stereoisomeren Formen und ihre pharmazeutisch unbedenklichen Säureadditionssalze.

Besonders hervorgehoben seien Verbindungen der allgemeinen Formel (I), in welcher

$R^1$ Wasserstoff oder einen geradkettigen, verzweigten oder cyclischen, gesättigten aliphatischen Kohlenwasserstoffrest bedeutet, der gegebenenfalls durch O, S, N, N-Alkyl oder NH unterbrochen ist und der gegebenenfalls substituiert ist durch Hydroxy, Alkoxy, Alkyl, Trifluormethyl, Halogen, Alkoxycarbonyl oder Dialkylamino, und

$R^2$ die für $R^1$ angegebene Bedeutung besitzt, wobei $R^2$ und $R^1$ gleich oder verschieden sein können, oder $R^2$ gemeinsam mit $R^1$ mit dem Stickstoffatom einen 5- bis 7-gliedrigen gesättigten oder ungesättigten Ring bildet, der gegebenenfalls 1 oder 2 weitere Heteroatome aus der Gruppe Sauerstoff, Schwefel oder Stickstoff enthält, wobei der Stickstoff gegebenenfalls durch Wasserstoff oder Alkyl substituiert ist, wobei dieser 5- bis 7-gliedrige Ring durch 1 bis 4 gleiche oder verschiedene Substituenten aus der Gruppe Alkyl, Halogen, Aryl, Aralkyl, Alkoxycarbonyl, Hydroxyalkyl, Alkoxyalkyl oder Trifluormethyl, substituiert sein kann,

A für eine gegebenenfalls durch Sauerstoff oder Stickstoff unterbrochene Kette aus 1-4 Kohlenstoffatomen steht, die gesättigt oder ungesättigt oder Teil eines 4- bis 7-gliedrigen Ringes sein kann und bei der jedes Stickstoff- bzw. Kohlenstoffatom durch Reste $R^1$ substituiert sein kann, die gleich oder verschieden sein können,

$R^3$ für einen Phenyl-, Furyl-, Naphthyl-, Thienyl-, Pyrimidyl-, Pyrazinyl-, Chinolyl-, Isochinolyl- oder Pyridylrest steht, welcher gegebenenfalls substituiert ist durch ein oder zwei gleiche oder verschiedene Substituenten aus der Gruppe Halogen, Nitro, Trifluormethyl, Alkyl, Alkoxy, Dialkylamino oder S-Alkyl wobei die genannten Alkyl- und Alkoxyreste jeweils 1 bis 4 Kohlenstoffatome enthalten,

$R^4$ und $R^5$ gleich oder verschieden sind und für Wasserstoff oder Alkyl mit 1 bis 4 Kohlenstoffatomen stehen und

$R^6$ und $R^7$ die für $R^1$ angegebene Bedeutung besitzen und mit $R^1$ gleich oder verschieden sind oder gemeinsam mit dem Stickstoffatom einen 5- bis 7-gliedrigen Ring bilden, der gegebenenfalls durch ein Sauerstoff- oder Stickstoffatom unterbrochen ist, wobei der Stickstoff gegebenenfalls durch Wasserstoff, Alkyl mit 1-4 Kohlenstoffatomen oder Benzyl substituiert ist,

sowie ihre stereoisomeren Formen der verschiedenen Enantiomeren, Diastereomeren und Z/E-Isomeren sowie ihre pharmazeutisch unbedenklichen Säureadditionssalze.

Die Herstellung der erfindungsgemäßen Imidazothiadiazolalkencarbonsäureamide der allgemeinen Formel (I) erfolgt, indem man

a) Carbonylverbindungen der allgemeinen Formel (II)

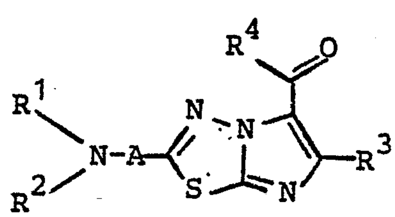

(II)

in welcher

A und $R^1$ bis $R^4$ die oben angegebene Bedeutung haben,

mit Phosphonatverbindungen der allgemeinen Formel (III)

$$R^{14}O-\underset{\overset{\|}{O}}{\overset{OR^{15}}{\overset{|}{P}}}-\underset{\overset{|}{R^5}}{CH}-CONR^6R^6 \qquad (III)$$

in welcher

$R^5$ bis $R^7$ die oben angegebene Bedeutung haben,

und

$R^{14}$ und $R^{15}$ für gegebenenfalls substituiertes Alkyl oder Aralkyl stehen,

in Gegenwart von starken Basen und in inerten organischen Lösungsmitteln bei Temperaturen zwischen -20 und 110°C umsetzt,

oder

b) Carbonylverbindungen der allgemeinen Formel (II) mit Acetamidderivaten der allgemeinen Formel (V)

$$R^5-CH_2-CONR^6R^7 \qquad (V)$$

in welcher

$R^5$, $R^6$ und $R^7$ die oben angegebene Bedeutung haben,

in Gegenwart von sauren oder basischen Katalysatoren und gegebenenfalls in Gegenwart von inerten organischen Lösungsmitteln bei Temperaturen zwischen 20 und 200°C umsetzt,

oder

c) Alkencarbonsäuren der allgemeinen Formel (VI)

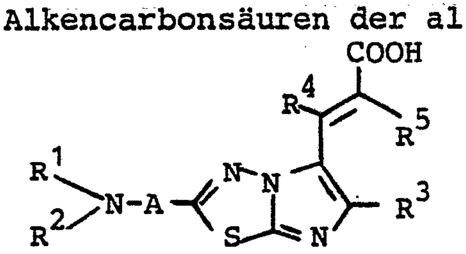

(VI)

in welcher

A und $R^1$ bis $R^5$ die oben angegebene Bedeutung haben,

mit Aminen der allgemeinen Formel (VII)

$$HNR^6R^7 \qquad (VII)$$

in welcher

$R^6$ und $R^7$ die oben angegebene Bedeutung haben,

gegebenenfalls nach Aktivierung der Carboxylgruppe über das entsprechende Säurechlorid (z.B. durch Thionylchlorid) in üblicher Weise in organischen inerten Lösungsmitteln bei Temperaturen zwischen 20 und 150°C amidiert,

oder

d) Imidazothiadiazol-alkencarbonsäureamide der allgemeinen Formel (VIII)

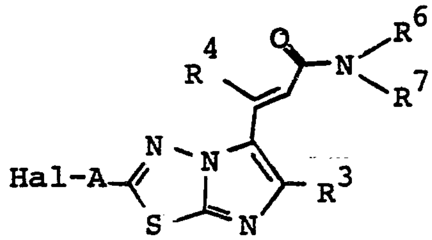

(VIII)

in welcher

A und $R^3$ bis $R^7$ die oben angegebene Bedeutung haben und

Hal für ein Element aus der Reihe Chlor, Brom oder Jod steht,

mit einem Amin der allgemeinen Formel $R^1R^2NH$, in welcher $R^1$ und $R^2$ die oben angegebene Bedeutung haben, in einem geeigneten Lösungsmittel bei Temperaturen von 0-150°C, gegebenenfalls in Gegenwart einer organischen oder anorganischen Hilfsbase umsetzt.

Je nach Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die

Antipoden als auch die Racemate und die Diastereomerengemische sind Gegenstand der vorliegenden Erfindung. Die Racemate lassen sich ebenso wie die Diastereomeren in bekannter Weise in die reinen Stereoisomeren trennen (vgl. E.L. Eliel, Stereochemistry of Carbon Compounds, McGraw Hill 1962).

Darüber hinaus sind für die erfindungsgemäßen Verbindungen infolge der Doppelbindung in der Seitenkette E/Z-Isomere möglich, die nach bekannten Verfahren hergestellt oder nach bekannten Verfahren ineinander überführt werden können. Als bekanntes Verfahren sei z.B. die Bestrahlung mit UV-Strahlen genannt.

Die als Ausgangsverbindungen einsetzbaren Carbonylverbindungen der allgemeinen Formel (II) sind bisher nicht bekannt, können aber in analoger Weise nach bekannten Methoden aus Imidazothiadiazolen der allgemeinen Formel (IX)

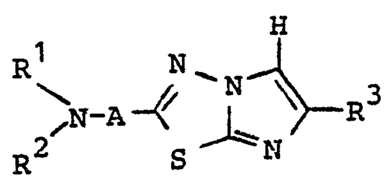

(IX)

in welcher

A und $R^1$ bis $R^3$ die oben angegebene Bedeutung haben,

hergestellt werden, indem man sie mit Dimethylformamid in Gegenwart von Phosphoroxychlorid bei Temperaturen von

20°C bis 170°C umsetzt, (vgl. z.B. L. Pentimalli et al., Boll. Sci. Fac. Chim. Ind. Bologna 23, 181 (1965); C.A. 63, 17848 e (1965), D. Bower et al., J. Chem. Soc. 1955, 2834, A. Hetzheim et al., Chem. Ber. 103, 3333 (1970), H. Beyer et al., Z. Chem. 2, 152 (1962) und S. Kano, Yagukagu Zasski 92, 935 (1972)).

Die als Ausgangsverbindungen einsetzbaren Alkencarbonsäuren der allgemeinen Formel (VI) sind ebenfalls neu. Sie können nach bekannten Methoden hergestellt werden, indem man

a) Carbonylverbindungen der allgemeinen Formel (II) mit Phosphonatverbindungen der allgemeinen Formel (X)

$$R^{14}O-\overset{\overset{OR^{15}}{|}}{\underset{\underset{O}{\|}}{P}}-\underset{\underset{R^5}{|}}{CH}-COOR^{16} \qquad (X)$$

in welcher

$R^5$,$R^{14}$ und $R^{15}$ die oben angegebene Bedeutung haben und

$R^{16}$ für H, Trialkyl oder Triarylsilyl oder für einen Rest der gleichen Bedeutung wie $R^{14}$ steht,

in Gegenwart von starken Basen in inerten organischen Lösungsmitteln zu den Alkencarbonsäurederivaten umsetzt und diese dann mit Säuren oder Laugen zu den freien Carbonsäuren verseift (vgl. W.S. Wadsworth et al., JACS 83, 1733 (1961)), oder

b) Carbonylverbindungen der allgemeinen Formel (II), in welcher $R^1$ bis $R^3$ die oben angegebene Bedeutung haben und $R^4$ gleich Wasserstoff ist (Aldehyde), mit Malonsäuren der allgemeinen Formel

$$R^5\text{-}CH(COOH)_2$$

in welcher

$R^5$ die oben angegebene Bedeutung hat,

oder mit Meldrumsäure in Gegenwart von inerten organischen Lösungsmitteln gegebenenfalls in Gegenwart von Kondensationsmitteln umsetzt (vgl. G. Jones, Org. Reactions, Vol. 15, S. 204 ff).

Die als Ausgangsverbindungen einsetzbaren Imidazothiadiazol-alkencarbonsäureamide der allgemeinen Formel (VIII) können aus entsprechenden Hydroxy-alkylimidazothiadiazol-alkencarbonsäureamiden der allgemeinen Formel (XI)

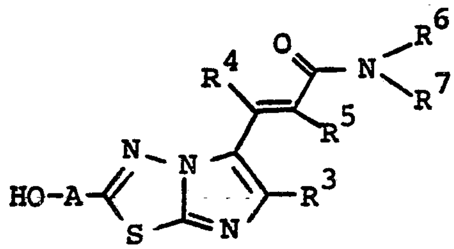

(XI)

durch Umsetzung mit einem geeigneten Halogenierungsmittel (z.B. Halogeniden des 3- und 5-wertigen Phosphors, Säurechloriden der schwefligen Säure oder Säurechloriden aliphatischer Carbonsäuren, vorzugsweise der Oxalsäure) in Gegenwart von Dimethylformamid oder anderen Carbonsäureamiden in geeigneten organischen Lösungsmitteln bei Temperaturen von -20 bis 120°C erhalten werden. Die Imidazothiadiazol-alkencarbonsäureamide der allgemeinen Formel (XI) können wie in DE-OS 3 043 158 beschrieben synthetisiert werden.

Die bei der Durchführung des erfindungsgemäßen Herstellungsverfahrens eingesetzten Phosphonatverbindungen der allgemeinen Formel (III) sind bekannt oder können nach bekannten Methoden hergestellt werden (vgl. I. Shahak et al. Isr. J. Chem. 7, 585 (1969)).

Als starke Basen zur Verwendung bei der Durchführung der Verfahrensvariante a) seien beispielhaft genannt:

Alkalihydride, wie Natriumhydrid, Kaliumhydrid, Lithiumhydrid und Alkalialkoholate wie Natriumethylat, Kaliumethylat oder Kaliummethylat oder Alkalimetallalkyle wie Methyllithium oder Butyllithium.

Die bei der Durchführung der Verfahrensvariante b) eingesetzten Acetamidderivate der allgemeinen Formel (V) sind bekannt oder können nach bekannten Methoden hergestellt werden (vgl. a) Brit. Pat. 715 896 (1954); C.A. <u>49</u>, 13290d (1955); b) DE-PS 1 142 859 (1960); C.A. <u>59</u>, 7377c (1963)).

Bei dieser Verfahrensvariante b) werden vorzugsweise saure oder basische Katalysatoren eingesetzt, von denen beispielhaft genannt seien: basische Amine wie Dialkylamine, Piperidin oder Pyridin oder anorganische Säuren, insbesondere Salzsäure oder Kondensationsmittel wie Carbonsäureanhydride.

Die gemäß Verfahrensvariante c) eingesetzten Alkensäuren der allgemeinen Formel (VI) sind bisher noch nicht bekannt geworden, lassen sich aber nach den oben angegebenen Verfahren in an sich bekannter Weise herstellen. Die für die Umsetzung mit Aminen zweckmäßige Aktivierung der freien Carboxylgruppe erfolgt vorzugsweise über das entsprechende Säurehalogenid, insbesondere über das entsprechende Säurechlorid unter Verwendung von Halogenidbildnern, wie z.B. Thionylchlorid, Phosphortrichlorid und Phosphorpentachlorid.

Bei allen erfindungsgemäßen Verfahren können als Verdünnungsmittel die üblichen inerten organischen Lösungsmittel eingesetzt werden. Hierzu gehören vorzugsweise Ether wie Diethylether, Glykolether wie Glykoldimethylether, Dioxan, Tetrahydrofuran oder Alkohole

wie Methanol, Ethanol, Propanol, Butanol, Benzylalkohol oder Sulfoxide wie Dimethylsulfoxid, Basen wie Pyridin, Chinolin, Picolin oder Kohlenwasserstoffe wie Benzol, Toluol, Xylol sowie Dimethylformamid.

Bei der Herstellung der Alkensäuren der allgemeinen Formel (VI) werden für die Verseifung der korrespondierenden Ester als Basen vorzugsweise benutzt: Alkalimetallhydroxide wie Natriumhydroxid, Kaliumhydroxid oder Erdalkalihydroxide wie Bariumhydroxid oder Calciumhydroxid.

Bei der Herstellung über die Aldehyde der allgemeinen Formel (II) mit $R^4$ = H mit Malonsäuren der allgemeinen Formel $R^5$-$CH(COOH)_2$ werden als Kondensationsmittel vorzugsweise verwendet:
Pyridin, substituierte Pyridinderivate wie Dialkylaminopyridine, Chinolin, Isochinolin, Dialkylamine wie Dimethylamin, Dibutylamin, Pyrrolidin, Piperidin und ähnliche stickstoffhaltige organische Basen.

Die als Zwischenprodukte verwendbaren Carbonylverbindungen der allgemeinen Formel (II) und die Alkencarbonsäuren der allgemeinen Formel (VI) sowie die Verfahren zu ihrer Herstellung sind ebenfalls Gegenstand der Erfindung.

Die erfindungsgemäßen Verbindungen zeichnen sich überraschenderweise durch starke biologische Wirkungen aus. Insbesondere besitzen sie ausgeprägte diuretische

und saluretische Wirkungen und können daher als Diuretika, Saluretika und Antihypertensiva verwendet werden. Bei Tierversuchen an Mäusen, Ratten bzw. Hunden zeigt sich, daß die erfindungsgemäßen Verbindungen bei oraler Applikation bereits bei Dosierungen unter 10 mg/kg eine ausgeprägte diuretische und saluretische Wirkung bei gleichzeitiger guter Verträglichkeit besitzen. Bei Kenntnis des Standes der Technik konnten diese vorteilhaften Eigenschaften nicht erwartet werden.

Die überraschenden und vorteilhaften Wirkungen der erfindungsgemäßen Verbindungen lassen sich nach den folgenden Testmethoden ermitteln:

A) Antihypertensive Wirkung an Ratten

Die Blutdruckwirkung wird ermittelt an Goldblatthochdruckratten nach Breuninger, H.: Methoden zur unblutigen Messung des Blutdruckes an Kleintieren, Arzneimittelforsch. 6, 222-225 (1965).

B) Diuretische Wirkung an Ratten

Nüchterne männliche Ratten im Gewicht von 150 bis 250 g (SPF, Wistar, je n = 4 Paare) werden mit 10 ml/kg p.o. Tylose-Suspension (0,5 %) als Kontrollen bzw. mit 100 mg/kg p.o. Prüfsubstanz in 10 ml/kg p.o. Tylose-Suspension mittels Schlundsonde behandelt. Die Tiere werden in Stoffwechselkäfige verbracht und die Harn- und Elektrolytausscheidung über 6 Stunden bestimmt ($Na^+$ und $K^+$ Bestimmung: IL-Flammenphotometer).

C) Diuretische Wirkung an Hunden

Bei nüchternen, wachen weiblichen Beagle-Hunden wird die Harnblase katheterisiert und die Harn- und Elektrolytausscheidung über 180 Minuten (fraktioniert über jeweils 30 Minuten) bestimmt.
Die Tiere erhalten während dieser Zeit über eine Dauerinfusion eine Elektrolytlösung intravenös und am Beginn des Versuches die Prüfsubstanz in 1 ml/kg Tylose-Suspension (0,5 %) oral zugeführt.
Der Harn wird auf $Na^+$, $K^+$, Chlor, Bicarbonat und pH analysiert.

D) Diuretische Wirkung an Mäusen

Nüchterne männliche SPF-Mäuse im Gewicht von 20 bis 25 g (n = 6x3 Tiere/Käfig) erhalten 100 ml/kg Tylose-Suspension (0,5 %) als Kontrollen, bzw. 100 mg/kg Prüfsubstanz in Tylose-Suspension oral zugeführt.
Die Ausscheidung von Harn, $Na^+$ und $K^+$ bzw. Harnsäure wird über 2 und 4 Stunden in Stoffwechselkäfigen bestimmt.

E) Phenolrot-Retentionstest an Ratten

Zum Nachweis von uricosurischer Wirksamkeit wird an wachen, nüchternen männlichen Ratten (SPF-Wistar, Gewicht 180 bis 250 g) der Einfluß von erfindungsgemäßen Verbindungen auf den Phenolrot-Blutspiegel dargestellt. Nach der Methode von Kreppel, E. (Med. exp. 1 (1959), 285-289) erhalten je 8 Tiere 75 mg/kg Phenolrot in 5 ml/kg Kochsalzlösung intraperitoneal, nachdem ihnen 30 Minuten vorher entweder 10 mg/kg Tylose-Suspension (0,5 %) als Kontrollen oder 100 mg/kg Prüfsubstanz in Tylose-Suspension verabreicht worden war. Plasma wird durch Punktion des retroorbitalen Venenplexus 30, 60 und 120 Minuten nach Gabe von Phenolrot, bzw. 60, 90 und 150 Minuten nach Substanzgabe gewonnen, mit NaOH versetzt und die Extinktion bei 546 nm im Photometer (Eppendorf) bestimmt.

Eine potentielle uricosurische Wirksamkeit liegt vor, wenn die Extinktionswerte signifikant höher als in der Kontrollgruppe sind.

Die neuen erfindungsgemäßen Verbindungen sind als Arzneimittel verwendbare Substanzen. Sie bewirken bei oraler oder parenteraler Anwendung eine Steigerung der Wasser- und Salzausscheidung und können daher zur Behandlung oedematöser und hypertoner Zustände und zur Ausschwemmung toxischer Substanzen dienen.

Darüber hinaus können die Verbindungen bei akutem Nierenversagen eingesetzt werden. Insbesondere zeigen sie auch eine vorteilhafte uricosurische Wirkung.

Die neuen Verbindungen können in bekannter Weise in die üblichen Formulierungen übergeführt werden, wie Tabletten, Kapseln, Dragees, Pillen, Granulate, Sirupe, Emulsionen, Suspensionen und Lösungen, unter Verwendung inerter, nichttoxischer, pharmazeutisch geeigneter Trägersubstanzen oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den unten angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral.

Für den Fall der parenteralen Anwendung können Lösungen der Wirkstoffe unter Verwendung geeigneter flüssiger

Trägermaterialien eingesetzt werden. Als besonders vorteilhaft für den Fall der parenteralen Anwendung hat sich die Tatsache herausgestellt, daß die erfindungsgemäßen Verbindungen in Wasser gut lösliche Salze zu bilden vermögen. Derartige Salze können auch für die orale Anwendung der erfindungsgemäßen Verbindungen eine erhöhte Bedeutung besitzen, indem sie die Resorption je nach Wunsch beschleunigen oder verzögern.

Im allgemeinen hat es sich als vorteilhaft erwiesen, bei parenteraler Applikation Mengen von etwa 0,05 bis 100 mg/kg, vorzugsweise etwa 0,1 bis 10 mg/kg Körpergewicht pro Tag zur Erzielung wirksamer Ergebnisse zu verabreichen. Bei oraler Applikation beträgt die Dosierung etwa 0,1 bis 500 mg/kg, vorzugsweise 0,5 bis 100 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen.

Diese Angaben gelten sowohl für die Anwendung der erfindungsgemäßen Verbindungen in der Veterinär- als auch in der Humanmedizin.

Das nachfolgende Beispiel 1 erläutert die Herstellung der erfindungsgemäß als Ausgangssubstanzen verwendeten Imidazothiadiazole der allgemeinen Formel (IX)

Das Beispiel 2 erläutert die Herstellung der erfindungsgemäß verwendeten Carbonylverbindungen der allgemeinen Formel (II).

Die dann folgenden Beispiele erläutern die Herstellung der erfindungsgemäßen Imidazolalkensäureamide der allgemeinen Formel (I) (Tabelle 1).

Beispiel 1

Herstellung von Imidazothiadiazolen der allgemeinen Formel (IX)

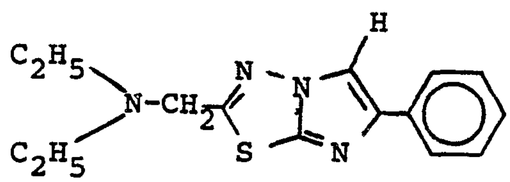

2-Diethylaminomethyl-6-phenyl-imidazo/2,1-b/-1,3,4-thiadiazol

18,6 g 2-Amino-5-diethylaminomethyl-1,3,4-thiadiazol (0,1 mol) und 19,9 g ω-Brom-acetophenon in 70 ml DMF werden 3 Stunden auf 150°C erwärmt. Die erkaltete Reaktionsmischung wird mit 150 ml Wasser versetzt und mit 20 %iger Natronlauge neutralisiert. Der Niederschlag wird abgesaugt. Nach Umkristallisation aus Acetonitril verbleiben 11,5 g (40,2 %) vom Schmelzpunkt 138°C.

Beispiel 2

Herstellung von Carbonylverbindungen der allgemeinen Formel (II)

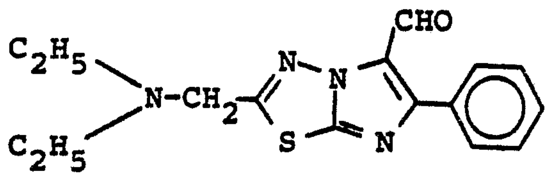

2-Diethylaminomethyl-6-phenyl-imidazo/2,1-b/-1,3,4-thiadiazol-5-carbaldehyd

2,3 ml Phosphoroxychlorid (0,025 mol) werden bei 0-5°C zu 20 ml Dimethylformamid getropft. Danach gibt man 5,7 g 2-Diethylaminomethyl-6-phenyl-imidazo-/2,1-b/-1,3,4-thiadiazol (0,02 mol) zu und erwärmt 2 Stunden auf 100°C. Nach dem Abkühlen wird mit 50 ml Wasser versetzt, mit 20 %iger Natronlauge neutralisiert und mit Ether extrahiert.
Nach dem Einengen wird das Rohprodukt aus Ether/Petrolether umkristallisiert.
Ausbeute: 3,0 g (48 %) Schmelzpunkt: 60°C

Beispiele für die Herstellung von Aminoalkyl-imidazothiadiazol-alkencarbonamiden der allgemeinen Formel (I)

Beispiel 3

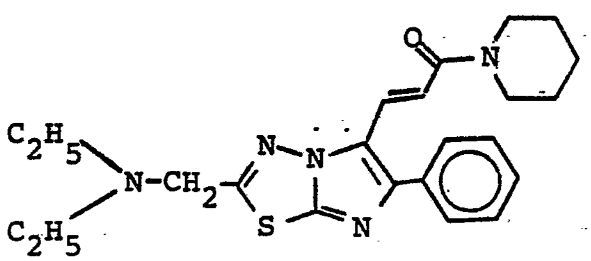

ß-(2-Diethylaminomethyl-6-phenyl-imidazo-/2,1-b/-1,3,4-thiadiazol-5-yl)-E-propenoyl-piperidid

Zu 0,33 g Natriumhydrid 80 % (0,011 Mol; mit Petrolether entölt) in 100 ml Toluol (wasserfrei) fügt man 2,9 g Diethylphosphonoessigsäurepiperidid (0,011 mol) und erwärmt auf 60°C, bis die Wasserstoffentwicklung beendet ist. Bei Raumtemperatur bringt man 2,86 g 2-Diethylaminomethyl-6-phenyl-imidazo-/2,1-b/-1,3,4-thiadiazol-5-carbaldehyd hinzu und rührt eine Stunde bei 50°C. Nach dem Abkühlen schüttelt man zweimal mit Natriumcarbonat-Lösung 10 %, trocknet über Natriumsulfat und engt ein.
Das Rohprodukt wird aus Isopropanol umkristallisiert.
Ausbeute: 2,75 g (65 %) Schmelzpunkt: 176°C.
Das Hydrochlorid schmilzt bei 210 °C.

Beispiel 4

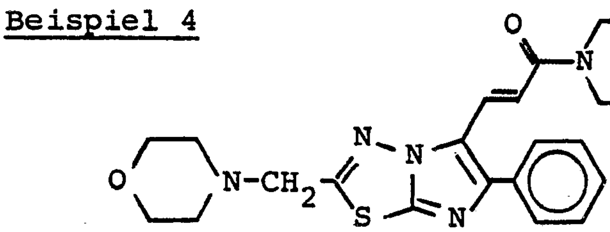

ß-(2-Morpholinomethyl-6-phenyl-imidazo-/2,1-b/-1,3,4-thiadiazol-5-yl)-E-propenoyl-morpholid

Zu einer Lösung von 1 g Dimethylformamid in 50 ml Dichlormethan tropft man bei 5-10°C 1,3 g Oxalylchlorid. Nach 30 Minuten fügt man 3,7 g ß-(2-hydroxymethyl-6-phenyl-imidazo-/2,1-b/-1,3,4-thiadiazol-5-yl)-E-propenoyl-morpholid zu und erwärmt 30 Minuten unter

Rückfluß. Dann fügt man 2 g Morpholin zu und erwärmt 1 Stunde zum Rückfluß. Nach dem Ausschütteln mit Wasser und Trocknen über Natriumsulfat engt man ein und kristallisiert den Rückstand aus Chloroform/Ether um. Ausbeute: 2,4 g (55 %) Schmelzpunkt: 207-9°C.

In analoger Weise wurden die Verbindungen gemäß Tabelle 1 synthetisiert.

Tabelle 1

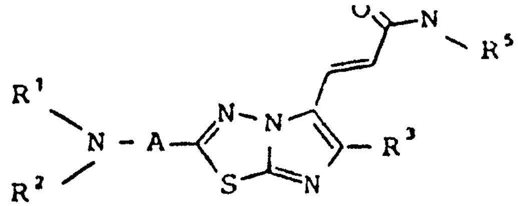

| Beispiel Nr. | A | $R^1$ | $R^2$ | $R^3$ | $R^4$ | $R^5$ | Ausbeute | Schmp. |
|---|---|---|---|---|---|---|---|---|
| 5 | $-CH_2-$ | $C_2H_5$ | $C_2H_5$ | $C_6H_5$ | $C_2H_5$ | $C_2H_5$ | 73% | 104°C |
| 6 | $-CH_2-$ | $C_2H_5$ | $C_2H_5$ | $C_6H_5$ | 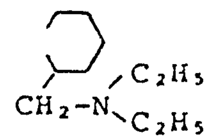 | | 30% | 124-5°C |
| 7 | $-CH_2-$ | $C_2H_5$ | $C_2H_5$ | $C_6H_5$ | 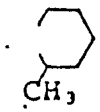 | | 35% | 138-40°C |
| 8 | $-CH_2-$ | $C_2H_5$ | $C_2H_5$ | $C_6H_5$ | 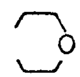 | | 61% | 181°C |
| 9 | $-CH_2-$ | $C_2H_5$ | $C_2H_5$ | $C_6H_5$ | 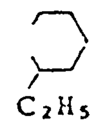 | | 50% | 106-7°C |
| 10 | $-CH_2-$ | $C_6H_5$ | $CH_3$ | $C_6H_5$ | 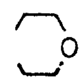 | | 41% | 155°C |
| 11 | $-CH_2-$ | 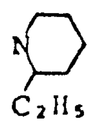 | | $C_6H_5$ | 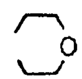 | | 28% | 160°C |

Tabelle 1 (Fortsetzung)

| Beispiel Nr. | A | $R^1$ $R^2$ | | $R^3$ | $R^4$ $R^5$ | | Ausbeute | Schmp. |
|---|---|---|---|---|---|---|---|---|
| 12 | $-CH_2-$ | 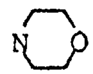 | | $C_6H_5$ | 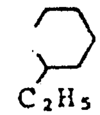 | | 56% | 194°C |
| 13 | $-CH_2-$ | 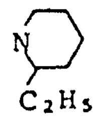 | | $C_6H_5$ | 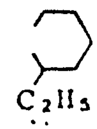 | | 40% | 117-9°C |
| 14 | $-CH_2-$ | 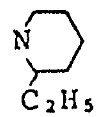 | | $C_6H_5$ | $C_2H_5$ | $C_2H_5$ | 38% | 129-30°C |
| 15 | $-CH_2-$ | 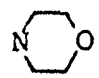 | | $C_6H_5$ | $C_2H_5$ | $C_2H_5$ | 70% | 173-4°C |
| 16 | $-CH_2-$ | $C_6H_5$ | $CH_3$ | $C_6H_5$ | $C_2H_5$ | $C_2H_5$ | 23% | 125°C |

Nach den erfindungsgemäßen Verfahren können auch die nachstehenden Verbindungen dargestellt werden.

ß-[2-Dimethylaminomethyl-6-phenyl[2.1-b]-1,3,4-thiadiazol-5-yl]-E-propenoyl-piperidid

ß-[2-Dimethylaminomethyl-6-phenyl[2.1-b]-1,3,4-thiadiazol-5-yl]-E-propenoyl-morpholid

ß-[2-Dimethylaminomethyl-6-phenyl[2.1-b]-1,3,4-thiadiazol-5-yl]-E-propenoyl-2-ethyl-piperid

ß-[2-Dimethylaminomethyl-6-phenyl[2.1-b]-1,3,4-thiadiazol-5-yl]-E-propenoyl-diethylamid

ß-[2-Dimethylaminomethyl-6-phenyl[2.1-b]-1,3,4-thiadiazol-5-yl]-E-propenoyl-pyrrolidid

ß-[2-Dimethylaminomethyl-6-phenyl[2.1-b]-1,3,4-thiadiazol-5-yl]-E-propenoyl-diisopropylamid

Patentansprüche

1. Imidazothiadiazolalkencarbonsäureamide der allgemeinen Formel (I)

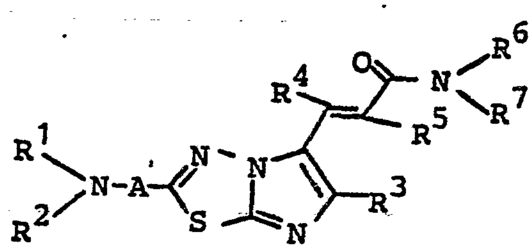

(I)

in welcher

$R^1$ Wasserstoff, Aryl oder einen aliphatischen Kohlenwasserstoffrest bedeutet, der gegebenenfalls durch O, S, N, N-Alkyl, NH, N-Aryl oder N-Aralkyl unterbrochen ist und der gegebenenfalls substituiert ist durch Hydroxy, Alkoxy, Alkyl, Trifluormethyl, Halogen, Phenyl, Alkoxycarbonyl oder Dialkylamino, wobei die beiden Alkylreste gegebenenfalls gemeinsam mit dem N-Atom einen 5- bis 7-gliedrigen Ring bilden, der gegebenenfalls durch ein Heteroatom aus der Gruppe O, S, NH oder N-Alkyl unterbrochen ist, und wobei diese vorgenannten Alkyl- und Phenylreste ihrerseits gegebenenfalls substituiert sind durch Halogen, Trifluormethyl, Alkyl, Aryl, Aralkyl, Alkoxy, Alkylmercapto oder $SO_2$-Alkyl, und

$R^2$ die für $R^1$ angegebene Bedeutung besitzt, wobei $R^2$ und $R^1$ gleich oder verschieden sein können, oder $R^2$ gemeinsam mit $R^1$ mit dem Stickstoffatom einen 3- bis 8-gliedrigen gesättigten oder ungesättigten Ring bildet, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl, Aryl oder Aralkyl substituiert ist, wobei dieser 3- bis 8-gliedrige Ring durch 1 bis 4 gleiche oder verschiedene Substituenten aus der Gruppe Alkyl, Halogen, Aryl, Aralkyl, Alkoxycarbonyl, Hydroxyalkyl, Alkoxyalkyl oder Trifluormethyl substituiert sein kann, oder wobei dieser Ring mit einem aromatischen Ring kondensiert sein kann,

A für eine gegebenenfalls durch 1 oder 2 Heteroatome aus der Gruppe Sauerstoff, Schwefel und Stickstoff unterbrochenen Kette aus 1-4 Kohlenstoffatomen steht, die gesättigt oder ungesättigt oder Teil eines 3- bis 8-gliedrigen Ringes sein kann und bei der jedes Stickstoff- und Kohlenstoffatom durch Reste $R^1$ substituiert sein kann, welche gleich oder verschieden sein können,

$R^3$ die für $R^1$ angegebene Bedeutung besitzt und mit $R^1$ gleich oder verschieden ist, oder für Furyl, Phenyl, Thienyl, Pyrimidyl, Pyrazinyl, Chinolinyl, Isochinolinyl oder Pyridyl steht, wobei die Ringe gegebenenfalls substituiert sind durch

1, 2 oder 3 gleiche oder verschiedene Substituenten aus der Gruppe Alkyl, Aryl, Alkoxy, Halogen, Nitro, Trifluormethyl, $SO_n$-Alkyl (n = 0, 1 oder 2) oder $NR^9R^{10}$, wobei $R^9$ und $R^{10}$ die oben angegebene Bedeutung von $R^1$ und $R^2$ besitzen,

$R^4$ für Wasserstoff, Trifluormethyl oder Alkyl steht,

$R^5$ für Wasserstoff, Alkyl, Cyano, Halogen, Nitro, $SO_n$-Alkyl (n = 0, 1 oder 2) oder $CXR^8$ steht, wobei X = O oder S bedeutet, und $R^8$ die oben angegebene Bedeutung von $R^1$ besitzt, und

$R^6$ und $R^7$ jeweils die für $R^1$ angegebene Bedeutung besitzen und mit $R^1$ gleich oder verschieden sind oder gemeinsam mit dem Stickstoffatom einen 3- bis 8-gliedrigen gesättigten oder ungesättigten Ring bilden, der gegebenenfalls ein oder zwei weitere Heteroatome aus der Gruppe Sauerstoff, Schwefel oder Stickstoff enthält, wobei der Stickstoff gegebenenfalls durch Wasserstoff, Alkyl, Aryl oder Aralkyl substituiert ist, und wobei dieser 3- bis 8-gliedrige Ring gegebenenfalls durch 1, 2, 3 oder 4 gleiche oder verschiedene Substituenten aus der Gruppe Alkyl, Aryl, Aralkyl, Halogen, Hydroxy, Alkoxycarbonyl, Hydroxyalkyl, Alkoxyalkyl,

Alkoxy oder Trifluormethyl substituiert ist und wobei dieser Ring mit einem gegebenenfalls durch ein bis drei dieser Substituenten substituierten aromatischen Ring kondensiert sein kann,

sowie ihre stereoisomeren Formen der verschiedenen Enantiomeren, Diastereomeren und E/Z-Isomeren sowie ihre pharmazeutisch unbedenklichen Säureadditionssalze.

2. Verbindungen der allgemeinen Formel (I) gemäß Anspruch 1, in welcher

$R^1$ Wasserstoff, Aryl oder einen aliphatischen Kohlenwasserstoffrest bedeutet, der gegebenenfalls durch O, S, N, N-Alkyl oder NH unterbrochen ist und der gegebenenfalls substituiert ist durch Hydroxy, Alkoxy, Alkyl, Trifluormethyl, Halogen, Alkoxycarbonyl oder Dialkylamino, wobei die beiden Alkylreste gegebenenfalls gemeinsam mit dem N-Atom einen 5- bis 7-gliedrigen Ring bilden, der gegebenenfalls durch ein Heteroatom aus der Gruppe O, S, NH oder N-Alkyl unterbrochen ist, und wobei diese vorgenannten Alkyl- und Phenylreste ihrerseits gegebenenfalls substituiert sind durch Halogen, Trifluormethyl, Alkyl, Alkoxy, Alkylmercapto oder $SO_2$-Alkyl, und

$R^2$ die für $R^1$ angegebene Bedeutung besitzt, wobei $R^2$ und $R^1$ gleich oder verschieden sein können, oder $R^2$ gemeinsam mit $R^1$ mit dem Stickstoffatom einen 5- bis 7-gliedrigen gesättigten oder ungesättigten Ring bildet, der gegebenenfalls 1 oder 2 weitere Heteroatome aus der Gruppe Sauerstoff, Schwefel oder Stickstoff enthält, wobei der Stickstoff gegebenenfalls durch Wasserstoff oder Alkyl substituiert ist, wobei dieser 5- bis 7-gliedrige Ring durch 1 bis 4 gleiche oder verschiedene Substituenten aus der Gruppe Alkyl, Halogen, Aryl, Aralkyl, Alkoxycarbonyl, Hydroxyalkyl, Alkoxyalkyl oder Trifluormethyl substituiert sein kann,

A für eine gegebenenfalls durch ein Heteroatom aus der Gruppe Sauerstoff, Schwefel und Stickstoff unterbrochene Kette aus 1-4 Kohlenstoffatomen steht, die gesättigt oder ungesättigt oder Teil eines 4- bis 7-gliedrigen Ringes sein kann und bei der jedes Stickstoff- und Kohlenstoffatom durch Reste $R^1$ substituiert sein kann, die gleich oder verschieden sein können,

$R^3$ für einen Phenyl-, Naphthyl-, Furyl-, Thienyl-, Pyrimidyl-, Pyrazinyl-, Chinolyl-, Isochinolyl- oder Pyridylrest steht, welcher gegebenenfalls substituiert ist durch 1 bis 3 gleiche oder verschiedene Substituenten aus der Gruppe Halogen, Nitro, Trifluormethyl, Alkyl, Alkoxy, Dialkylamino oder $SO_n$-Alkyl (n = 0, 1 oder 2), wobei die genannten Alkyl- und Alkoxyreste jeweils 1-4 Kohlenstoffatome enthalten,

$R^4$ für Wasserstoff, Trifluormethyl oder Alkyl steht,

$R^5$ für Wasserstoff, Alkyl, Cyano, Halogen oder $CXR^8$ steht, wobei X = O oder S bedeutet, und $R^8$ die oben angegebene Bedeutung von $R^1$ besitzt, und

$R^6$ und $R^7$ jeweils die für $R^1$ angegebene Bedeutung besitzen und mit $R^1$ gleich oder verschieden sind oder
gemeinsam mit dem Stickstoffatom einen 5- bis 7-gliedrigen gesättigten oder ungesättigten Ring bilden, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl mit 1 bis 4 Kohlenstoffatomen, Phenyl oder Benzyl substituiert ist,

sowie ihre stereoisomeren Formen der verschiedenen Enantiomeren, Diastereomeren und E/Z-Isomeren sowie ihre pharmazeutisch unbedenklichen Säureadditionssalze.

3. Verbindungen der allgemeinen Formel (I) gemäß Anspruch 1, in welcher

$R^1$ Wasserstoff oder einen geradkettigen, verzweigten oder cyclischen, gesättigten aliphatischen Kohlenwasserstoffrest bedeutet, der gegebenenfalls durch O, S, N, N-Alkyl oder NH unterbrochen ist und der gegebenenfalls substituiert ist durch Hydroxy, Alkoxy, Alkyl, Trifluormethyl, Halogen, Alkoxycarbonyl oder Dialkylamino, und

$R^2$ die für $R^1$ angegebene Bedeutung besitzt, wobei $R^2$ und $R^1$ gleich oder verschieden sein können, oder $R^2$ gemeinsam mit $R^1$ mit dem Stickstoffatom einen 5- bis 7-gliedrigen gesättigten oder ungesättigten Ring bildet, der gegebenenfalls 1 oder 2 weitere Heteroatome aus der Gruppe Sauerstoff, Schwefel oder Stickstoff enthält, wobei der Stickstoff gegebenenfalls durch Wasserstoff oder Alkyl substituiert ist, wobei dieser 5- bis 7-gliedrige Ring durch 1 bis 4 gleiche oder verschiedene Substituenten aus der Gruppe Alkyl, Halogen, Aryl, Aralkyl, Alkoxycarbonyl, Hydroxyalkyl, Alkoxyalkyl oder Trifluormethyl, substituiert sein kann,

A für eine gegebenenfalls durch Sauerstoff oder Stickstoff unterbrochene Kette aus 1-4 Kohlenstoffatomen steht, die gesättigt oder ungesättigt oder Teil eines 4- bis 7-gliedrigen Ringes sein kann und bei der jedes Stickstoff- bzw. Kohlenstoffatom durch Reste $R^1$ substituiert sein kann, die gleich oder verschieden sein können,

$R^3$ für einen Phenyl-, Furyl-, Naphthyl-, Thienyl-, Pyrimidyl-, Pyrazinyl-, Chinolyl-, Isochinolyl- oder Pyridylrest steht, welcher gegebenenfalls

substituiert ist durch ein oder zwei gleiche oder verschiedene Substituenten aus der Gruppe Halogen, Nitro, Trifluormethyl, Alkyl, Alkoxy, Dialkylamino oder S-Alkyl wobei die genannten Alkyl- und Alkoxyreste jeweils 1 bis 4 Kohlenstoffatome enthalten,

$R^4$ und $R^5$ gleich oder verschieden sind und für Wasserstoff oder Alkyl mit 1 bis 4 Kohlenstoffatomen stehen und

$R^6$ und $R^7$ die für $R^1$ angegebene Bedeutung besitzen und mit $R^1$ gleich oder verschieden sind oder gemeinsam mit dem Stickstoffatom einen 5- bis 7-gliedrigen Ring bilden, der gegebenenfalls durch ein Sauerstoff- oder Stickstoffatom unterbrochen ist, wobei der Stickstoff gegebenenfalls durch Wasserstoff, Alkyl mit 1-4 Kohlenstoffatomen oder Benzyl substituiert ist,

sowie ihre stereoisomeren Formen der verschiedenen Enantiomeren, Diastereomeren und Z/E-Isomeren sowie ihre pharmazeutisch unbedenklichen Säureadditionssalze.

4. ß-(2-Diethylaminomethyl-6-phenyl-imidazo-/2,1-b/-1,3,4-thiadiazol-5-yl/-E-propenoyl-piperidid und sein Hydrochlorid.

5. Verfahren zur Herstellung von Imidazothiadiazolalkencarbonsäureamiden gemäß Anspruch 1 bis 3, dadurch gekennzeichnet, daß man

a) Carbonylverbindungen der allgemeinen Formel (II)

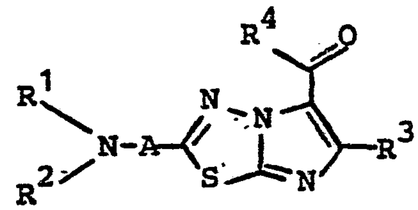

(II)

in welcher

A für eine gegebenenfalls durch 1 oder 2 Heteroatome aus der Gruppe Sauerstoff, Schwefel und Stickstoff unterbrochenen Kette aus 1-4 Kohlenstoffatomen steht, die gesättigt oder ungesättigt oder Teil eines 3- bis 8-gliedrigen Ringes sein kann und bei der jedes Stickstoff- und Kohlenstoffatom durch Reste $R^1$ substituiert sein kann, welche gleich oder verschieden sein können,

$R^1$ Wasserstoff, Aryl oder einen aliphatischen Kohlenwasserstoffrest bedeutet, der gegebenenfalls durch O, S, N, N-Alkyl, NH, N-Aryl oder N-Aralkyl unterbrochen ist und der gegebenenfalls substituiert ist durch Hydroxy, Alkoxy, Alkyl, Trifluormethyl, Halogen, Phenyl, Alkoxycarbonyl oder Dialkylamino, wobei die beiden Alkylreste gegebenenfalls gemeinsam mit dem N-Atom einen 5- bis 7-gliedrigen Ring bilden, der gegebenenfalls durch ein Heteroatom aus der Gruppe O, S, NH oder N-Alkyl

unterbrochen ist, und wobei diese vorgenannten Alkyl- und Phenylreste ihrerseits gegebenenfalls substituiert sind durch Halogen, Trifluormethyl, Alkyl, Aryl, Aralkyl, Alkoxy, Alkylmercapto oder $SO_2$-Alkyl, und

$R^2$ die für $R^1$ angegebene Bedeutung besitzt, wobei $R^2$ und $R^1$ gleich oder verschieden sein können, oder $R^2$ gemeinsam mit $R^1$ mit dem Stickstoffatom einen 3- bis 8-gliedrigen gesättigten oder ungesättigten Ring bildet, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl, Aryl oder Aralkyl substituiert ist, wobei dieser 3- bis 8-gliedrige Ring durch 1 bis 4 gleiche oder verschiedene Substituenten aus der Gruppe Alkyl, Halogen, Aryl, Aralkyl, Alkoxycarbonyl, Hydroxyalkyl, Alkoxyalkyl oder Trifluormethyl substituiert sein kann, oder wobei dieser Ring mit einem aromatischen Ring kondensiert sein kann,

$R^3$ die für $R^1$ angegebene Bedeutung besitzt und mit $R^1$ gleich oder verschieden ist, oder für Furyl, Phenyl, Thienyl, Pyrimidyl, Pyrazinyl, Chinolinyl, Isochinolinyl oder Pyridyl steht, wobei die Ringe gegebenenfalls substituiert sind durch 1, 2 oder 3

gleiche oder verschiedene Substituenten aus der Gruppe Alkyl, Aryl, Alkoxy, Halogen, Nitro, Trifluormethyl, $SO_n$-Alkyl (n = 0, 1 oder 2) oder $NR^9R^{10}$, wobei $R^9$ und $R^{10}$ die oben angegebene Bedeutung von $R^1$ und $R^2$ besitzen,

$R^4$ für Wasserstoff, Trifluormethyl oder Alkyl steht,

mit Phosphonatverbindungen der allgemeinen Formel (III)

$$R^{14}O-\underset{\overset{\|}{O}}{\overset{\overset{OR^{15}}{|}}{P}}-\underset{\overset{|}{R^5}}{CH}-CONR^6R^7 \qquad (III)$$

in welcher

$R^5$ für Wasserstoff, Alkyl, Cyano, Halogen, Nitro, $SO_n$-Alkyl (N = 0, 1 oder 2) oder $CXR^8$ steht, wobei X = O oder S bedeutet, und $R^8$ die oben angegebene Bedeutung von $R^1$ besitzt, und

$R^6$ und $R^7$ jeweils die für $R^1$ angegebene Bedeutung besitzen und mit $R^1$ gleich oder verschieden sind oder gemeinsam mit dem Stickstoffatom einen 3- bis 8-gliedrigen gesättigten oder ungesättigten Ring bilden, der gegebenenfalls ein oder zwei weitere Heteroatome aus der Gruppe Sauerstoff, Schwefel oder Stickstoff enthält, wobei der Stickstoff gegebenenfalls durch Wasserstoff, Alkyl, Aryl oder Aralkyl substituiert ist, und wobei dieser 3- bis 8-gliedrige Ring gegebenenfalls durch 1,2,3 oder 4 gleiche oder verschiedene Substituenten aus der Gruppe Alkyl, Aryl, Aralkyl, Halogen, Hydroxy, Alkoxycarbonyl, Hydroxyalkyl, Alkoxyalkyl, Alkoxy oder Trifluormethyl substituiert ist und wobei dieser Ring mit einem gegebenenfalls durch ein bis drei dieser Substituenten substituierten aromatischen Ring kondensiert sein kann, und

$R^{14}$ und $R^{15}$ für gegebenenfalls substituiertes Alkyl oder Aralkyl stehen,

in Gegenwart von starken Basen und in inerten organischen Lösungsmitteln bei Temperaturen zwischen -20 und 110°C umsetzt,

b) Carbonylverbindungen der allgemeinen Formel (II) mit Acetamidderivaten der allgemeinen Formel (V)

$$R^5\text{-}CH_2\text{-}CONR^6R^7 \qquad (V)$$

in welcher

$R^5$,$R^6$ und $R^7$ die oben angegebene Bedeutung haben,

in Gegenwart von sauren oder basischen Katalysatoren und gegebenenfalls in Gegenwart von inerten organischen Lösungsmitteln bei Temperaturen zwischen 20 und 200°C umsetzt, oder

c) Alkencarbonsäuren der allgemeinen Formel (VI)

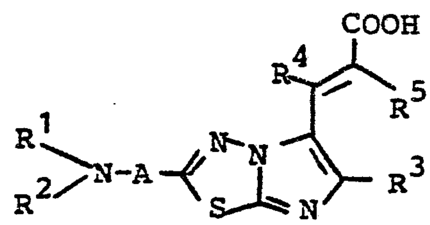

(VI)

in welcher

A und $R^1$ bis $R^5$ die oben angegebene Bedeutung haben,

mit Aminen der allgemeinen Formel (VII)

$$HNR^6R^7 \qquad (VII)$$

in welcher

$R^6$ und $R^7$ die angegebene Bedeutung haben,

gegebenenfalls nach Aktivierung der Carboxylgruppe über das entsprechende Säurechlorid in üblicher Weise in organischen inerten Lösungsmitteln bei Temperaturen zwischen 20 und 150°C amidiert,
oder

d) Imidazothiadiazol-alkencarbonsäureamide der allgemeinen Formel (VIII)

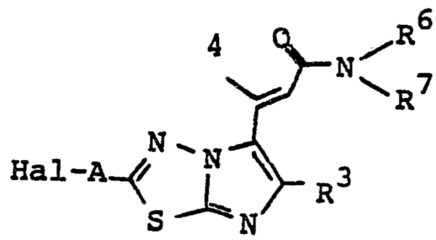

(VIII)

in welcher

A und $R^3$ bis $R^7$ die angegebene Bedeutung haben und

Hal für ein Element aus der Reihe Chlor, Brom oder Jod steht,

mit einem Amin der allgemeinen Formel $R^1R^2NH$, in welcher $R^1$ und $R^2$ die oben angegebene Bedeutung haben, in einem geeigneten Lösungsmittel bei Temperaturen von 0-150°C gegebenenfalls in Gegenwart einer organischen oder anorganischen Hilfsbase umsetzt,

und sie gegebenenfalls nach üblichen Methoden in Säureadditionssalze überführt.

6. Verbindungen gemäß Anspruch 1 bis 3 zur Verwendung bei der Bekämpfung von Erkrankungen.

0141072

7. Verbindungen gemäß Anspruch 1 bis 3 zur Verwendung als Antihypertensiva, Diuretika und Urikosurika.

8. Verfahren zur Herstellung von Arzneimitteln, dadurch gekennzeichnet, daß man Verbindungen gemäß Anspruch 1 bis 3 gegebenenfalls unter Verwendung üblicher Hilfs- und Trägerstoffe in eine geeignete Applikationsform überführt.

9. Carbonylverbindungen der allgemeinen Formel (II)

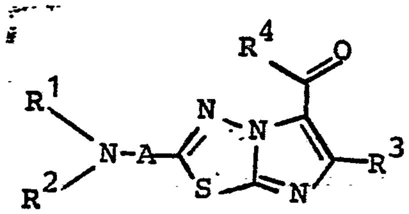

(II)

in welcher

A für eine gegebenenfalls durch 1 oder 2 Heteroatome aus der Gruppe Sauerstoff, Schwefel und Stickstoff unterbrochenen Kette aus 1-4 Kohlenstoffatomen steht, die gesättigt oder ungesättigt oder Teil eines 3- bis 8-gliedrigen Ringes sein kann und bei der jedes Stickstoff- und Kohlenstoffatom durch Reste $R^1$ substituiert sein kann, welche gleich oder verschieden sein können,

$R^1$ Wasserstoff, Aryl oder einen aliphatischen Kohlenwasserstoffrest bedeutet, der gegebenenfalls durch O, S, N, N-Alkyl, NH, N-Aryl oder N-Aralkyl unterbrochen ist und der gegebenenfalls substituiert ist durch Hydroxy, Alkoxy, Alkyl, Trifluormethyl, Halogen, Phenyl, Alkoxycarbonyl oder Dialkylamino, wobei die beiden Alkylreste gegebenenfalls gemeinsam mit dem N-Atom einen 5- bis 7-gliedrigen Ring bilden, der gegebenenfalls durch ein Heteroatom aus der Gruppe O, S, NH oder N-Alkyl unterbrochen ist, und wobei diese vorgenannten Alkyl- und Phenylreste ihrerseits gegebenenfalls substituiert sind durch Halogen, Trifluormethyl, Alkyl, Aryl, Aralkyl, Alkoxy, Alkylmercapto oder $SO_2$-Alkyl, und

$R^2$ die für $R^1$ angegebene Bedeutung besitzt, wobei $R^2$ und $R^1$ gleich oder verschieden sein können, oder $R^2$ gemeinsam mit $R^1$ mit dem Stickstoffatom einen 3- bis 8-gliedrigen gesättigten oder ungesättigten Ring bildet, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl, Aryl oder Aralkyl substituiert ist,

wobei dieser 3- bis 8-gliedrige Ring durch 1 bis 4 gleiche oder verschiedene Substituenten aus der Gruppe Alkyl, Halogen, Aryl, Aralkyl, Alkoxycarbonyl, Hydroxyalkyl, Alkoxyalkyl oder Trifluormethyl substituiert sein kann, oder wobei dieser Ring mit einem aromatischen Ring kondensiert sein kann,

$R^3$ die für $R^1$ angegebene Bedeutung besitzt und mit $R^1$ gleich oder verschieden ist, oder für Furyl, Phenyl, Thienyl, Pyrimidyl, Pyrazinyl, Chinolinyl, Isochinolinyl oder Pyridyl steht, wobei die Ringe gegebenenfalls substituiert sind durch 1, 2 oder 3 gleiche oder verschiedene Substituenten aus der Gruppe Alkyl, Aryl, Alkoxy, Halogen, Nitro, Trifluormethyl, $SO_n$-Alkyl (n = 0, 1 oder 2) oder $NR^9R^{10}$, wobei $R^9$ und $R^{10}$ die oben angegebene Bedeutung von $R^1$ und $R^2$ besitzen,

$R^4$ für Wasserstoff, Trifluormethyl oder Alkyl steht.

10. Alkencarbonsäuren der allgemeinen Formel (VI)

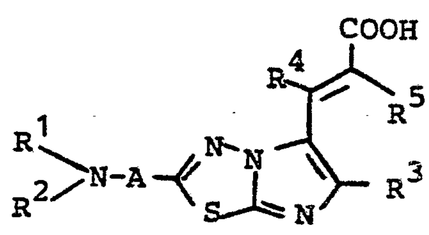

(VI)

in welcher

$R^1$ Wasserstoff, Aryl oder einen aliphatischen Kohlenwasserstoffrest bedeutet, der gegebenenfalls durch O, S, N, N-Alkyl, NH, N-Aryl oder N-Aralkyl unterbrochen ist und der gegebenenfalls substituiert ist durch Hydroxy, Alkoxy, Alkyl, Trifluormethyl, Halogen, Phenyl, Alkoxycarbonyl oder Dialkylamino, wobei die beiden Alkylreste gegebenenfalls gemeinsam mit dem N-Atom einen 5- bis 7-gliedrigen Ring bilden, der gegebenenfalls durch ein Heteroatom aus der Gruppe O, S, NH oder N-Alkyl unterbrochen ist, und wobei diese vorgenannten Alkyl- und Phenylreste ihrerseits gegebenenfalls substituiert sind durch Halogen, Trifluormethyl, Alkyl, Aryl, Aralkyl, Alkoxy, Alkylmercapto oder $SO_2$-Alkyl, und

$R^2$ die für $R^1$ angegebene Bedeutung besitzt, wobei $R^2$ und $R^1$ gleich oder verschieden sein können, oder $R^2$ gemeinsam mit $R^1$ mit dem Stickstoffatom einen 3- bis 8-gliedrigen gesättigten oder ungesättigten Ring bildet, der gegebenenfalls 1 oder 2 weitere Heteroatome aus der Gruppe Sauerstoff, Schwefel oder Stickstoff enthält, wobei der Stickstoff gegebenenfalls durch Wasserstoff, Alkyl, Aryl oder Aralkyl substituiert ist, wobei dieser 3- bis 8-gliedrige Ring durch 1 bis 4 gleiche oder verschiedene Substituenten aus der Gruppe Alkyl, Halogen, Aryl, Aralkyl, Alkoxycarbonyl, Hydroxyalkyl, Alkoxyalkyl oder Trifluormethyl substituiert sein kann, oder wobei dieser Ring mit einem aromatischen Ring kondensiert sein kann,

A für eine gegebenenfalls durch 1 oder 2 Heteroatome aus der Gruppe Sauerstoff, Schwefel und Stickstoff unterbrochenen Kette aus 1-4 Kohlenstoffatomen steht, die gesättigt oder ungesättigt oder Teil eines 3- bis 8-gliedrigen Ringes sein kann und bei der jedes Stickstoff- und Kohlenstoffatom durch Reste $R^1$ substituiert sein kann, welche gleich oder verschieden sein können,

$R^3$ die für $R^1$ angegebene Bedeutung besitzt und mit $R^1$ gleich oder verschieden ist, oder für Furyl, Phenyl, Thienyl, Pyrimidyl, Pyrazinyl, Chinolinyl, Isochinolinyl oder Pyridyl steht, wobei die Ringe gegebenenfalls substituiert sind durch 1, 2 oder 3 gleiche oder verschiedene Substituenten aus der Gruppe Alkyl, Aryl, Alkoxy, Halogen, Nitro, Trifluormethyl, $SO_n$-Alkyl ($n = 0$, 1 oder 2) oder $NR^9R^{10}$, wobei $R^9$ und $R^{10}$ die oben angegebene Bedeutung von $R^1$ und $R^2$ besitzen,

$R^4$ für Wasserstoff, Trifluormethyl oder Alkyl steht,

$R^5$ für Wasserstoff, Alkyl, Cyano, Halogen, Nitro, $SO_n$-Alkyl ($n = 0$, 1 oder 2) oder $CXR^8$ steht, wobei X = O oder S bedeutet, und $R^8$ die oben angegebene Bedeutung von $R^1$ besitzt.